(19) Europäisches Patentamt
European Patent Office
Office européen des brevets

(11) Publication number: **0 440 098 B1**

# (12) EUROPEAN PATENT SPECIFICATION

(45) Date of publication of patent specification: **05.04.95**

(51) Int. Cl.6: **C07C 327/32**, A61K 31/265

(21) Application number: **91100947.0**

(22) Date of filing: **25.01.91**

## (54) Optically active derivative of d-2-(6-methoxy-2-naphthyl)-propionic acid and pharmaceutical compositions containing it.

(30) Priority: **29.01.90 IT 1918490**

(43) Date of publication of application:
**07.08.91 Bulletin 91/32**

(45) Publication of the grant of the patent:
**05.04.95 Bulletin 95/14**

(84) Designated Contracting States:
**AT BE CH DE DK ES FR GB GR IT LI LU NL SE**

(56) References cited:
**EP-B- 0 124 925**

(73) Proprietor: **FARMA RESA S.r.l.**
**Via Como, 5**
**I-22063 Cantu'**
**Como (IT)**

(72) Inventor: **Reiner, Valentina**
**Via Roma, 3**
**I-22010 Carate Urio (IT)**
Inventor: **Sarda, Caterina**
**Via Mentana, 23**
**I-22100 Como (IT)**

(74) Representative: **Dragotti, Gianfranco et al**
**SAIC BREVETTI s.r.l.**
**Viale Bianca Maria, 15**
**I-20122 Milano (IT)**

EP 0 440 098 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an optically active isomer of a derivative of d-2-(6-methoxy-2-naphthyl)-propionic acid, namely of the active principle known as Naproxen; the invention relates as well to pharmaceutical compositions containing the said derivative.

Naproxen is known since a number of years and is included among the substances having anti-inflammatory, analgesic and anti-pyretic activity: its main therapeutical use is the treatment of rheumatoid arthritis and of other degenerative forms having phlogistic features.

In turn alpha-mercaptopropionylglycine is a compound known as well, possessing it too anti-inflammatory activity besides the mucolytic activity.

The derivatives obtained by combining said two active principles are the object of the European Patent No. 124.925, which thus claims compounds having the following general formula:

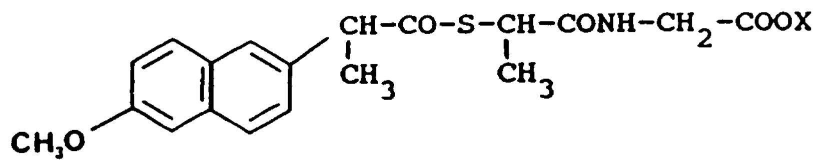

wherein X is hydrogen or a radical selected among the radical of organic or inorganic, non toxic and pharmaceutically acceptable bases, radicals of basic aminoacids and radicals of basic antibiotics.

The characteristics and properties of the above mentioned compounds in the racemic form, namely the (d,l) form, are given in the specification and in the examples of this European Patent.

It has been now found and is the object of the present invention that the (d,d) diastereoisomer of the compound directly obtained from Naproxen and alpha-metilpropionylglicyine has surprisingly specific properties which are superior not only with respect with the starting active principle, namely Naproxen, but also with respect to the corresponding (d,l) racemic compound.

This greater activity over Naproxen is mainly seen in the lack of ulcerogenicity which is on the contrary the main drawback of Naproxen as regards the therapeutical use, whereas in comparison with the corresponding racemic compound the diastereoisomer of the present invention has greater analgesic activity.

Otherwise stated, the diastereoisomer of the present invention to the prevailing anti-inflammatory activity which is characteristics both of Naproxen and more remarkably of the derivatives being the subject of the European Patent 124.925, adds a specific analgesic activity which is definitely higher than that the of the corresponding racemic compound.

It is evident that the therapeutical use of these substances makes the symptomatic activity, namely the analgesic one, of the utmost importance.

As regards the preparation of the diastereoisomer of the present invention, reference is made to the specification and examples of European Patent 124.925, particularly examples 1a and 1b of this patent, which is herein included by reference.

Thus according to the process studied with respect to the present invention, the product of the reaction carried out between the acid chloride of d-2-(6-methoxy-2-naphthyl)-propionic acid and (2-mercapto-propionyl)-glycine in the presence of a base is poured in water, then an acidification is carried out with hydrochloric acid and the solid thus precipitated is filtered. For the separation of the desired (d,d) form the racemic one repeated fractionated crystallizations are carried out, using ethyl acetate as the crystallization solvent, until a product having constant melting point (180-1 °C) is obtained.

The obtained compound has been subjected to elemental analysis with the following results:

Calculated for $C_{19}H_{21}NO_5S$: %C 60.78; %H 5,63; %N 3,73; %S 8,54

Found: %C 61.02; %H 5.59; %N3,71; %S 8.63.

The (d,d) diastereoisomer of the invention is a crystalline solid having $[alpha]_D^{20}$ + 201.01 (C=1, methanol).

It is soluble in ethanol, methanol and acetone, a little soluble in hot ethyl acetate and insoluble in water.

By carrying out the TLC analysis with chloroform: glacial acetic acid: water (85:15:0.5) as the eluant system, a value $R_f$ = 0.75 is obtained whereas the (d,l) diastereoisomer has $R_f$ = 0.70.

The already mentioned properties of the diastereoisomer according to the invention have been confirmed by the pharmacologycal tests which are hereinafter shortly reported (the compound of the invention is indicated in abbreviated form has d-d Nxtio whereas the other diastereoisomer is abbreviated as

d-l Nxtio).

As regards the anti-inflammatory activity the standard tests by means of carrageenan induced oedema and dextran induced oedema were performed whereas for the analgesic activity the writhing test and the Randall-Selitto test have been carried out.

1. Carrageenan induced oedema in the rat

Grups of 8 rats (Wistar-Charles River) have been orally administered with:
(i) vehicle alone (0.5% tragacanth);
(ii) d-d Nxtio at a constant volume of 24 ml/kg in the same vehicle;
(iii) d-l Nxtio at constant volume of 25 ml/kg in the same vehicle.

The table 1 hereinafter reports the administration dosages.

TABLE 1

| GROUP | TREATMENT VEHICLE | DOSE mg/kg p.o. |
|---|---|---|
| 1 | vehicle | -- |
| 2 | | 6.25 |
| 3 | | 12.5 |
| 4 | d-d Nxtio | 25.0 |
| 5 | | 50.0 |
| 6 | | 6.25 |
| 7 | | 12.5 |
| 8 | d-l Nxtio | 25.0 |
| 9 | | 50.0 |

Fourty five minutes later each rat received an injection of 0.1 ml of a 1% w/v suspension of carrageenan in sterile 0.9% saline, beneath the plantar aponeurosis of the left hind paw.

The volume of the paw was measured (in arbitrary units) before and 1.5, 3 and 6 hours after the irritant injection.

The results are reported in the table 2, from which it can be observed not only that both diastereoisomers administered by oral route induce a significant inhibition of the carrageenan induced oedema, but also that d-l isomer is slightly less active than the d-d isomer.

## TABLE 2

**Effects of oral administration of d-d Nxtio and d-l Nxtio on a carrageenan-induced oedema in the rat**

| GROUP | TREATMENT | DOSE mg/Kg p.o. | MEAN INCREASE IN PAW VOLUME | | | % INHIBITION OF SWELLING | | |
|---|---|---|---|---|---|---|---|---|
| | | | 1.5 | 3 | 6 | 1.5 | 3 | 6 |
| 1 | vehicle | | 7.9 +/- 0.58 | 12.1 +/- 0.52 | 13.4 +/- 0.76 | - | - | - |
| 2 | d-d Nxtio | 6.25 | 3.7 +/- 0.59 | 5.5 +/- 0.57 | 8.1 +/- 0.44 | 53.2 | 54.5 | 39.6 |
| 3 | | 12.5 | 4.7 +/- 0.95 | 8.9 +/- 0.90 | 8.7 +/- 1.12 | 40.5 | 26.4 | 35.1 |
| 4 | | 25.0 | 4.3 +/- 0.59 | 8.3 +/- 0.95 | 9.8 +/- 0.86 | 45.6 | 31.4 | 26.9 |
| 5 | | 50.0 | 5.0 +/- 0.94 | 8.4 +/- 0.76 | 8.4 +/- 0.86 | 36.7 | 30.6 | 37.3 |
| 6 | d-l Nxtio | 6.25 | 4.1 +/- 0.61 | 6.2 +/- 0.62 | 8.7 +/- 0.55 | 48.1 | 48.7 | 35.0 |
| 7 | | 12.5 | 4.9 +/- 0.92 | 9.3 +/- 1.10 | 9.4 +/- 1.12 | 37.9 | 23.1 | 29.8 |
| 8 | | 25.0 | 4.6 +/- 0.70 | 8.9 +/- 1.2 | 10.2 +/- 0.81 | 41.7 | 26.4 | 23.8 |
| 9 | | 50.0 | 5.6 +/- 0.92 | 8.7 +/- 0.8 | 9.8 +/- 0.84 | 29.1 | 28.0 | 26.8 |

2. Dextran induced oedema in the rat.

This test has been carried out like the preceeding one, except that instead of carrageenan 0.1 ml of a 6% suspension w/v of dextran in the same sterile saline solution where injected.

The measurement of the paw volume (in milliliters) was carried out before the injection and at 0.5, 1 and 2.5 hours after the injection.

The results reported in the table 3 confirm the anti-inflammatory activity of both isomers, with a slight superiority of d-d isomer.

## TABLE 3

**Effects of oral administration of d-d Nxtio and d-l Nxtio on a dextran-induced oedema in the rat**

| GROUP | TREATMENT | DOSE mg/Kg p.o. | MEAN INCREASE IN PAW VOLUME | | | % INHIBITION OF SWELLING | | |
|---|---|---|---|---|---|---|---|---|
| | | | 0.5 | 1 | 2.5 | 0.5 | 1 | 2.5 |
| 1 | vehicle | - | 1.09 +/- 0.05 | 1.28 +/- 0.04 | 1.08 +/- 0.06 | - | - | - |
| 2 | d-d Nxtio | 6.25 | 1.03 +/- 0.04 | 1.00 +/- 0.03 | 0.72 +/- 0.04 | 5.5 | 21.9 | 33.3 |
| 3 | | 12.5 | 1.12 +/- 0.06 | 1.16 +/- 0.04 | 0.83 +/- 0.06 | 0 | 9.4 | 23.2 |
| 4 | | 25.0 | 1.09 +/- 0.02 | 1.11 +/- 0.04 | 0.81 +/- 0.03 | 0 | 13.3 | 25.0 |
| 5 | | 50.0 | 1.24 +/- 0.05 | 0.90 +/- 0.04 | 0.73 +/- 0.02 | 0 | 29.7 | 32.4 |
| 6 | d-l Nxtio | 6.25 | 1.04 +/- 0.08 | 1.03 +/- 0.07 | 0.85 +/- 0.07 | 4.6 | 19.5 | 21.3 |
| 7 | | 12.5 | 1.11 +/- 0.04 | 1.11 +/- 0.08 | 0.79 +/- 0.08 | 0 | 13.3 | 26.8 |
| 8 | | 25.0 | 1.12 +/- 0.06 | 1.14 +/- 0.06 | 0.80 +/- 0.06 | 0 | 11.0 | 26.0 |
| 9 | | 50.0 | 1.20 +/- 0.05 | 1.10 +/- 0.04 | 0.9 +/- 0.04 | 0 | 14.0 | 16.6 |

3. Writhing test in the rat.

Groups of 10 rats (Wistar-Charles River) were dosed orally with either vehicle (0.5% tragacanth) or test compounds (d-d and X d-l Nxtio) at a costant volume of 10 ml/kg according to the treatment table 1.

Forty-five minutes later each rat received an intraperitoneal injection of 1.0 ml of 1% solution of acetic acid. They were then placed in individual cages and the number of writhes elicited by each rat in the following 25 minute period was recorded.

The results of this test are reported in the table 4, from which it can be observed that both diastereoisomers cause a dose dependent inhibition, with a marked superiority of the isomer d-d of the order of 20%.

## TABLE 4

### Analgesic activities of orally administered compounds in the writhing test - individual animal data

| TREATMENT | ORAL DOSE (mg/Kg) | NUMBER OF WRITHES/25 MIN FOR ANIMAL No. | | | | | | | | | | MEAN WRITHING SCORE (+/- s.e.) |
|---|---|---|---|---|---|---|---|---|---|---|---|---|
| | | 1 | 2 | 3 | 4 | 5 | 6 | 7 | 8 | 9 | 10 | |
| Vehicle | - | 49 | 54 | 40 | 65 | 43 | 48 | 23 | 65 | 19 | 57 | 46.3 +/- 5.24 |
| d-d Nxtio | 50 | 4 | 10 | 16 | 4 | 24 | 4 | 8 | 6 | 3 | 1 | 8.0 +/- 2.36 |
| | 25 | 35 | 7 | 16 | 16 | 5 | 36 | 16 | 36 | 23 | 21 | 21.1 +/- 3.81 |
| | 12.5 | 42 | 61 | 23 | 25 | 39 | 48 | 38 | 43 | 9 | 7 | 33.5 +/- 5.74 |
| | 6.25 | 47 | 59 | 33 | 46 | 71 | 23 | 26 | 25 | 24 | 47 | 40.1 +/- 5.52 |
| d-l Nxtio | 50 | 7 | 12 | 10 | 8 | 24 | 7 | 11 | 5 | 9 | 0 | 9.3 +/- 1.95 |
| | 25 | 24 | 15 | 32 | 15 | 7 | 28 | 25 | 31 | 20 | 25 | 22.2 +/- 2.51 |
| | 12.5 | 56 | 65 | 31 | 37 | 44 | 52 | 35 | 41 | 25 | 10 | 39.6 +/- 5.03 |
| | 6.25 | 48 | 51 | 45 | 39 | 31 | 34 | 46 | 37 | 26 | 72 | 42.9 +/- 4.09 |

4. Randall-Selitto test in the rat

Male Wistar rats (70-90 g) were starved 18 hours prior to the commencement of the experiment but water was available ad libitum.

Each rat received an injection of 0.1 ml of a 20% suspension of Brewer's yeast in distilled water beneath the plantar aponeurosis of the left hind paw.

One hour later the pain thresholds of both the inflamed (yeast injected) and normal hind paw were measured using an analgesiometer designed by U. Basile; the animals were then dosed orally with vehicle (0.5% tragacanth) or test compounds at a constant dose volume of 10 ml/kg.

There were 10 rats in each drug-treated group and 20 rats in the vehicle-treated group.

The pain thresholds of the inflamed and normal hind paws were again measured at 1, 2 and 4 hours after dosing.

The results are reported in the table 5 from which it can be observed not only a peak of analgesic effect at two hours from the administration but also the superiority of the (d-d) isomer.

## TABLE 5

**Changes in pain threshold of inflamed and normal paws of rats receiving oral treatment with d-d Nxtio and d-l Nxtio**

| GROUP | TREATMENT | DOSE (mg/Kg) p.o. | MEAN CHANGE IN PAIN THRESHOLD (G +/- S.E.) FROM PRE-DOSE AT POST-DOSE TIME | | | | | |
|---|---|---|---|---|---|---|---|---|
| | | | 1 HOUR | | 2 HOURS | | 4 HOURS | |
| | | | INFLAMED PAW | NORMAL PAW | INFLAMED PAW | NORMAL PAW | INFLAMED PAW | NORMAL PAW |
| 1 | vehicle | - | -22.4 +/- 15.2 | -40.5 +/- 17.8 | -42.0 +/- 15.1 | -52.6 +/- 18.0 | -21.3 +/- 15.3 | -39.2 +/- 13.9 |
| 2 | d-d Nxtio | 6.25 | -27.2 +/- 30.8 | -12.8 +/- 17.8 | -42.2 +/- 21.9 | -17.7 +/- 18.2 | -32.7 +/- 15.5 | 34.1 +/- 17.4 |
| 3 | | 12.5 | 32.6 +/- 40.1 | -13.7 +/- 22.9 | 32.6 +/- 41.7 | -23.4 +/- 18.2 | 38.9 +/- 48.6 | 10.7 +/- 23.7 |
| 4 | | 25 | 53.2 +/- 29.9 | - 1.0 +/- 26.0 | 31.3 +/- 35.8 | -33.5 +/- 24.4 | 41.9 +/- 35.3 | 18.7 +/- 35.9 |
| 5 | | 50 | 48.3 +/- 27.9 | - 1.3 +/- 22.9 | 98.9 +/- 38.9 | -38.8 +/- 18.9 | 79.4 +/- 42.7 | 25.7 +/- 24.3 |
| 6 | d-l Nxtio | 6.25 | -21.6 +/- 30.9 | -14.3 +/- 20.0 | -38.4 +/- 18.9 | -24.5 +/- 25.3 | -23.1 +/- 18.4 | -10 +/- 25.5 |
| 7 | | 12.5 | 25.5 +/- 37.3 | -15.4 +/- 29.6 | 20.5 +/- 38.7 | -20.9 +/- 25.6 | 21.2 +/- 43.2 | -12 +/- 15.8 |
| 8 | | 25 | 40.2 +/- 31.4 | -10.8 +/- 27.5 | 23.2 +/- 34.6 | -31.5 +/- 21.9 | 29.6 +/- 38.1 | 19 +/- 37.7 |
| 9 | | 50 | 42.3 +/- 31.6 | - 1.4 +/- 10.3 | 60.2 +/- 45.7 | -38.4 +/- 22.8 | 52.1 +/- 19.1 | 18 +/- 22.7 |

At that point it is worth to note that the advantages achieved by the present invention must be evaluated as a function of the relevant advancement already achieved with the compounds of the European Patent 124.925 whereby the further improvement which is thus obtained is, in obviously relative terms, a result relevant as well and fully unforeseable.

Preliminary clinical investigations have been carried out, with the results hereinafter stated:

I. Gastric tolerability.

This investigation has been carried out at the University of Milan under the supervision of the professor Mauro Podda, and has been directed to evaluate the gastric tolerability of d-d Nxtio in comparison both with Naproxen and with the combination of Naproxen and thiopronine: the study has been carried out on 18

voluntary patients in the need of an analgesic and anti-inflammatory treatment.

The experimental protocol was that of the double blind trial, the patients being randomly selected and treated for 10 days at a daily dosage of two tablets and more particularly:

(i) for Nxtio tablets of 550 mg containing an amount of active compound corresponding to 337 mg of Naproxen;

(ii) for Naproxen, tablets of the drug named "Naprosyn" containing 500 mg of Naproxen;

(iii) for the patients treated with Naproxen + thiopronine, tablets were used having an overall weight of 550 mg and corresponding to 337 mg of Maproxen.

Each patient was subjected to endoscopy by optical fibers both before and after the treatment to assess the gastric tolerability.

According to the endoscopic results, in 3 patients (two treated with naproxen + thiopronine and one with naproxen only) antral erosion was observed, whereas in two patients treated with naproxen only the appearance of antral gastritis and a worsening of the duodenitis was respectively observed.

In only one patient treated with Nxtio a slight duodenal hyperemy was observed.

The conclusions drawn from the evaluation of the endoscopic oservations and from the analysis of possible undesirable side effects confirm that the compound of the invention is endowed with an optimum gastrointestinal tolerability.

II. Efficacy and tolerability of Nxtio in patients suffering from post-traumatic pain.

This investigation has been carried out under the supervision of the prof. E.Bottarelli of the Physiokinesitherapy Division of the Hospital G.Stuard of Parma (Italy).

50 voluntary patients suffering from algic symptomatology of post-traumatic origin have been treated according to an open type experimental protocol, in which Nxtio has been compared with Naproxen (as the drug sold on the Italian market as "Naprosyn").

Two tablets of Nxtio of the overall weight of 550 mg (corresponding to a content of Naproxen of 337 mg) and of Naprosyn (with a Naproxen content of 500 mg) were daily administered to the patients until the symptomatology disappeared but for no more than 7 days.

According to the results Nxtio has an optimum therapeutical efficacy against the pain and all clinical situations of traumatic origin; The very important feature assessed in this investigation is that by using Nxtio not only the patients recovered a satisfactory condition before the end of the treatment period, but that the action of Nxtio takes place earlier and in a more effective manner in comparison with that of the comparison drug (namely Naproxen). Of course this fact is very important when dealing with situations of acute symptomatology and moreover nothing in the prior art would suggest that this derivative of Naproxen would show such an activity (i.e.the analgesic one) and in a more favourable manner.

In this connection it is to be taken into account that in this investigation the patients were on the average about 70 years old,and thus more liable to side effects of gastrointestinal origin as induced from the treatment with an anti-inflammatory drug.

III.Efficacy and tolerability of Nxtio in patients with pre-menstrual syndrome.

This investigation has been carried out at the Endocrinology Division of the I.N.I. Institute of Grottaferrata (Rome - Italy) under the supervision of the Dr.Filippo Vita.

80 voluntary young women suffering from pre-menstrual syndrome have been treated with Nxtio in comparison with Naproxen ("Naprosyn") according to an open type experimental protocol.

The treatment has been carried out for 20 days, the patients being administered with 2 tablets per day.

According to the preliminary results Nxtio has a better therapeutical activity than the comparison drug.

IV.Efficacy and tolerability of Nxtio in patients affected from algic symptomatology of post-surgical origin.

This investigation is still in course at the Hospital of Legnano (Italy) under the supervision of the director.

The investigation has been carried out on 50 voluntary patients suffering from post-surgical pain, the patients being daily administered with two tablets of Nxtio or of Naprosyn according to an open type experimental protocol.

The administration period was dependent on the post-surgical behaviour of the patient, but never longer than 7 days.

From the analysis of the therapeutical results it has been assessed that Nxtio has a quicker and more relevant efficacy than the comparison drug, accompanied by a very good tolerability, as confirmed by the lack of undesirable side effects at the gastrointestinal level.

The pharmaceutical compositions according to the present invention comprise as the active ingredient the (d-d) diastereoisomer together with the standard vehicles and excipients.

In this connection reference is made to the disclosure of European Patent 124.925, which is here incorporated for reference.

The dosages of active principles shall be still those of the corresponding composition based on Naproxen whereby the therapeutical effect is obviously increased.

As a matter of fact in the clinical experiments being carried out the following unit dosages are used:

- Tablets and capsules with a content of 275 and 550 mg of Nxtio
- suppositories with the same unit dosages
- gel for topical treatment with a 10% b.w. content of active ingredient.

## Claims

**1.** S-[d-2-(6-methoxy-2-naphthyl)propionyl]d-2-mercaptopropionamido acetic acid.

**2.** Use of the diastereoisomer of claim 1 for the preparation of pharmaceutical compositions for the treatment of the inflammatory states and as analgesic.

**3.** Pharmaceutical composition in form suitable for the administration by oral, topycal, rectal route, useful as anti-inflammatory and analgesic drug, characterized by containing as the active ingredient, the diastereoisomer of claim 1.

## Patentansprüche

**1.** S-[d-2-(6-Methoxy-2-naphthyl)propionyl]d-2-mercaptopropionamidoessigsäure.

**2.** Verwendung des Diastereoisomers gemäß Anspruch 1 zur Herstellung von pharmazeutischen Zusammensetzungen zur Behandlung von Entzündungszuständen und als Analgetikum.

**3.** Pharmazeutische Zusammensetzung in einer zur Verabreichung auf oralem, topischem oder rektalem Weg geeigneten Form, die als Arzneimittel gegen Entzündungen und als Analgetikum verwendbar ist, dadurch gekennzeichnet, daß sie als Wirkstoff das Diastereoisomer gemäß Anspruch 1 enthält.

## Revendications

**1.** Acide S[d-2-(6-méthoxy-2-naphtyl)proprionyl] d-2-mercaptopropionamido acétique.

**2.** Utilisation du diastereoisomère selon la revendication 1 pour la préparation de compositions pharmaceutiques pour le traitement d'états inflammatoires et comme analgésiques.

**3.** Composition pharmaceutique de forme appropriée à l'administration par voie orale, entopique, rectale, utilisé en tant que produit anti-inflammatoire et analgésiques, caractérisé en ce qu'il comprend comme ingrédient actif le diastereoisomère selon la revendication 1.